(19) Europäisches Patentamt
European Patent Office
Office européen des brevets

(11) Veröffentlichungsnummer: **0 000 532**
**B1**

(12) # EUROPÄISCHE PATENTSCHRIFT

(45) Veröffentlichungstag der Patentschrift: **07.10.81**

(51) Int. Cl.³: **C 07 D 303/04**, C 07 D 301/14

(21) Anmeldenummer: **78100418.9**

(22) Anmeldetag: **18.07.78**

(54) **Verfahren zur Herstellung von Vinyloxiran.**

(30) Priorität: **29.07.77 DE 2734240**

(43) Veröffentlichungstag der Anmeldung: **07.02.79 Patentblatt 79/3**

(45) Bekanntmachung des Hinweises auf die Patenterteilung: **07.10.81 Patentblatt 81/40**

(84) Benannte Vertragsstaaten: **DE**

(56) Entgegenhaltungen:
**DE-A-2 262 970**
**FR-A-2 300 085**
**FR-A-2 309 551**
**FR-A-2 309 552**
**FR-A-2 369 273**
**GB-A-846 534**

(73) Patentinhaber: **BAYER AG, Zentralbereich Patente, Marken und Lizenzen, D-5090 Leverkusen 1, Bayerwerk (DE)**

(72) Erfinder: **Seifert, Hermann, Dr., Ruwergasse 4, D-5000 Köln 80 (DE)**
Erfinder: **Waldmann, Helmut, Dr., Carl-Rumpff-Strasse 59, D-5090 Leverkusen 1 (DE)**
Erfinder: **Schwerdtel, Wulf, Dr., Hegelstrasse 9, D-5090 Leverkusen 1 (DE)**
Erfinder: **Swodenk, Wolfgang, Dr., Auf dem Broich 5, D-5068 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 000 532 B1

# Verfahren zur Herstellung von Vinyloxiran

Die vorliegende Erfindung betrifft ein verbessertes Verfahren zur kontinuierlichen Herstellung von Vinyloxiran.

Vinyloxiran, das Monoepoxid des Butadiens, ist ein wichtiges technisches Zwischenprodukt, das aufgrund seines bifunktionellen Charakters eine breite Anwendung auf dem Gebiet der Polymere findet (F.C. Frostick, B. Phillips and P.S. Starcher, Journal of Am. Chem. Society, Vol 81, p. 3351 (1959)). Vinyloxiran gehört zur Verbindungsklasse der Epoxy-vinyl-monomeren, die dadurch gekennzeichnet sind, dass sie durch Reaktion des Epoxidringes in Polyäther umgewandelt werden können. Diese Polyäther enthalten dann freie, zur Vernetzung oder Copolymerisation mit anderen olefinischen Verbindungen geeignete Vinylgruppen. Es ist aber auch möglich, Vinyloxiran zunächst an der olefinischen Doppelbindung des Moleküls zu polymerisieren, so dass dann Polymere erhalten werden, die zur Weiterreaktion verwendbare Expoxid-Gruppierungen in der Kohlenwasserstoffkette aufweisen.

Entsprechend der potentiellen technischen Bedeutung des Vinyloxirans hat es in der Vergangenheit nicht an Versuchen gefehlt, einfache und wirtschaftliche Verfahren zur Herstellung von Vinyloxiran aufzufinden. Dies ist bislang jedoch nicht gelungen. Die Schwierigkeiten bei der Herstellung von Vinyloxiran bestehen zum einen in der Reaktionsführung der Umsetzung von Butadien mit dem Epoxidierungsreagenz, zum anderen in der Auftrennung der bei der Umsetzung anfallenden, Vinyloxiran enthaltenden Reaktionsgemische.

So haben Pummerer und Reindel (Chem. Berichte 66, 335–339 (1933)) bereits 1933 zur Herstellung von Vinyloxiran aus Butadien die Verwendung von Perbenzoesäure vorgeschlagen. Jedoch lagen die erzielten Ausbeuten lediglich bei 40–60%.

Auch die Herstellung von Vinyloxiran unter Verwendung einer 3% Peressigsäure enthaltenden ätherischen Lösung dieser Percarbonsäure verläuft, da die Ausbeute nur 50% beträgt, nicht zufriedenstellend (Pudovik, Ivanow, Journal of Gen. Chem. of USSR Vol 26, 3087 (1956) in engl. translation).

Wesentliche Verbesserungen konnten auch von F.C. Frostick et. al., (Journal of Am. Chem. Soc. 81, 3354 (1959)) nicht erzielt werden. Bei der Umsetzung von Butadien mit durch Oxidation von Acetaldehyd gewonnener Peressigsäure in organischen Lösungsmitteln wurde eine nur 56% betragende Ausbeute erreicht.

Mittels Oxidation von Acetaldehyd gewonnene Peressigsäure wird auch in der japanischen Patentschrift Nr. J 7-4046-284 als zur Herstellung von Vinyloxiran geeignetes Reagenz beschrieben. Es werden jedoch keine Ausbeuten angegeben.

Verfahren zur Herstellung von Vinyloxiran, die aus Acetaldehyd hergestellte Peressigsäure zur Epoxidation von Butadien verwenden, besitzen den grundsätzlichen Nachteil, dass sie als Hilfsstoff Acetaldehyd benötigen, und dass dieser Hilfsstoff das Verfahren nicht unverändert verlässt, womit eine Rückführung in das Verfahren praktisch ausgeschlossen ist. Zudem sind derartige Verfahren in der technischen Durchführung aufwendig, da eine zusätzliche Oxidationsstufe erforderlich ist. Ausserdem ist die Oxidation von Acetaldehyd oder anderen Aldehyden zu Peressigsäure bzw. Percarbonsäuren eine schwierig durchzuführende Reaktion, deren Sicherheitsrisiken bei der technischen Anwendung, bedingt durch die als Zwischenstufen auftretenden, hochexplosiven Aldehydperacrylate, nicht unerheblich sind (D. Swern, «Organic Peroxides», Vol. 1, S. 353, 354; Wiley-Interscience (1971)).

In der Britischen Patentschrift 735 974 (Union Carbide Corp.) wird vorgeschlagen, zur Epoxidation von Butadien zu Vinyloxiran Acetaldehyd- oder Propionaldehydmonoperacylat, ebenfalls gewonnen durch Oxidation der entsprechenden Aldehyde, einzusetzen.

Der Umsatz an Acetaldehydmonoperacetat beträgt bei der Reaktion mit Butadien nur 33%. Die zur Gewinnung von Vinyloxiran sich anschliessende destillative Aufarbeitung eines derartigen Reaktionsgemisches erfolgt in Anwesenheit der Perverbindung, was eine technische Anwendung wegen der bestehenden Explosionsgefahr ausschliesst.

Auch die Anwendung spezieller verfahrenstechnischer Massnahmen, wie sie in der Deutschen Patentschrift 1 216 306 zur Durchführung von Epoxidationen vorgeschlagen werden, bringen im Falle des Butadiens nicht den gewünschten Erfolg, wie dem Beispiel 3 dieser Patentschrift entnommen werden kann, wo die Epoxidation von Butadien nach diesem Verfahren mit Peressigsäure lediglich eine 68%ige Selektivität für Vinyloxiran erbringt.

In der US-Patentschrift 3 053 856 wird die Umsetzung von Acetonitril und Wasserstoffperoxid in Gegenwart von Butadien beschrieben, wobei gemäss Beispiel XII dieser Patentschrift Vinyloxiran als Hauptprodukt entsteht. Da jedoch bei diesem Verfahren das dem jeweils eingesetzten Nitril entsprechende Carbonsäureamid erhalten wird, das als Koppelprodukt zu betrachten ist und entweder einer geeigneten Verwendung zugeführt oder aber durch Dehydratisierung in das Nitril zurückverwandelt werden muss, ist eine technisch befriedigende Lösung des Problems der Vinyloxiranherstellung nicht gegeben.

Die Verwendung von Alkylhydroperoxiden, wie Cumol- und tert.-Butylhydroperoxid, zur Epoxidation von Dienen unter dem katalytischen Einfluss von Molybdän- oder Vanadinverbindungen ist von Sheng und Zajacek beschrieben worden (Journal of Organic Chemistry Vol 35, 6, 1839–1843 (1970)). Butadien kann nach diesem Verfahren mit 85%iger Selektivität in Vinyloxiran überführt werden. Das Alkylhydroperoxid R-OOH wird bei dieser Reaktion, wie aus Gleichung (1) ersichtlich, in den entsprechenden Alkohol R-OH

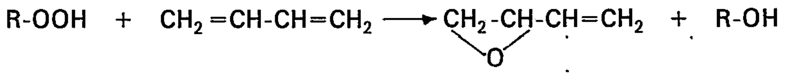 (1)

umgewandelt. Das Kohlenwasserstoffperoxid R-OOH wirkt also als Sauerstoffüberträger, so dass nach Abgabe des Peroxid-Sauerstoffs der entsprechende Alkohol als Koppelprodukt anfällt und häufig als unerwünschtes Nebenprodukt entfernt werden muss. Die technischen Einsatzmöglichkeiten eines solchen Verfahrens sind dementsprechend begrenzt, da nicht in jedem Falle das Nebenprodukt Alkohol verwertet werden kann.

Bei einem anderen Verfahren, das in der Belgischen Patentschrift 747 316 beschrieben ist, wird zur Herstellung von Vinyloxiran aus Butadien und Wasserstoffperoxid die Umsetzung in Gegenwart eines Zinn enthaltenden Katalysatorsystems durchgeführt. Neben dem Nachteil, dass zur Durchführung der Reaktion ein Lösungsmittel, wie beispielsweise n-Propanol, benötigt wird, das selbst wiederum mit bereits gebildetem Vinyloxiran reagieren kann, ist mit hohen Verlusten an Produkt zu rechnen, die dadurch bedingt sind, dass das aus dem eingesetzten Wasserstoffperoxid nach der Reaktion mit dem Butadien sich bildende Wasser mit Vinyloxiran unter Spaltung des Epoxidringes reagiert. Weiterhin ist die Abtrennung des Katalysators aus dem Reaktionsgemisch keine in technischem Masstab einfach zu lösende Aufgabe.

Ebenso zahlreich wie die Vorschläge, die gemacht wurden, um Verbesserungen für die Reaktionsführung bei der Epoxidation von Butadien zu Vinyloxiran zu erreichen, sind die Anstrengungen gewesen, die zur Bewältigung der bei einer technischen Durchführung der Auftrennung von Vinyloxiran enthaltenden Reaktionsgemischen sich stellenden Probleme unternommen worden sind. Diese Probleme bei der Aufarbeitung von Gemischen, die Vinyloxiran enthalten, sind im wesentlichen dadurch gegeben, dass der Epoxidring im Molekül des Vinyloxirans sehr leicht Nebenreaktionen eingeht, was zu teilweise erheblichen Verlusten an Vinyloxiran führt, und dass so die Wirtschaftlichkeit eines technischen Verfahrens zur Herstellung von Vinyloxiran nicht mehr gegeben ist. Die Gefahr des Auftretens von Produktverlusten besteht besonders dann, wenn die Gemische, aus denen Vinyloxiran abzutrennen ist, saure Komponenten enthalten.

Dies ist immer dann gegeben, wenn man Vinyloxiran aus Butadien und einer Percarbonsäure herstellt, da dann die der Percarbonsäure entsprechende Carbonsäure zwangsläufig im Reaktionsgemisch enthalten ist.

In der Britischen Patentschrift 852 097 wird für ein derartiges Reaktionsgemisch zur Trennung von Vinyloxiran und der Carbonsäure vorgeschlagen, die Carbonsäure, beispielsweise Essigsäure, dadurch zu entfernen, dass man zunächst eine Lösung von Vinyloxiran und der Carbonsäure in einem organischen, in Wasser nicht löslichen Lösungsmittel, wie beispielsweise chlorierten Kohlenwasserstoffen, herstellt und die Lösung danach mit Wasser oder einer wässrigen Lösung eines inerten Alkalimetallsalzes bei Temperaturen unter 15°C extrahiert. Dieses Extraktionsverfahren ist aufwendig, da es nicht nur ein zusätzliches Lösungsmittel erfordert, sondern auch eine nachfolgende Trennung des Wassers von der Carbonsäure notwendig macht, wenn die Carbonsäure wiedergewonnen werden soll. Die Gewinnung von Carbonsäuren aus wässrigen Lösungen auf destillativem Wege ist, wegen der Bildung azeotroper Gemische, eine nur mit erheblichem Aufwand zu bewältigende Aufgabe.

Der Umfang der für ein derartiges Extraktionsverfahren zur Gewinnung von Vinyloxiran technisch notwendigen Massnahmen ist der US-Patentschrift 3 019 234 zu entnehmen. Die nach Zusatz des in Wasser weitgehend unlöslichen Lösungsmittel resultierende, Vinyloxiran und die Carbonsäure enthaltende Lösung muss unterhalb 25°C mit Wasser oder einer wässrigen Lösung extrahiert werden. Der Extraktionsaufwand ist erheblich. Aus Beispiel 1 der obengenannten Patentschrift geht hervor, dass 10 theoretische Extraktionsstufen erforderlich sind. Zudem muss bei 0°C gearbeitet werden. Die durch dieses Verfahren zur Gewinnung von Vinyloxiran gegebene Problemlösung zur verlustfreien Trennung von Vinyloxiran und Carbonsäure ist nicht zufriedenstellend, da sich nicht unwesentliche Mengen an Vinyloxiran nach erfolgter Extraktion in der wässrigen Phase befinden.

In der Französischen Patentschrift 1 468 814 wird vorgeschlagen, das nach Durchführung der Epoxidierung des Butandiens anfallende, freie Carbonsäure – in diesem Falle Essigsäure – enthaltende Reaktionsgemisch, das daneben noch einen Essigsäureester als Lösungsmittel, Vinyloxiran und Butadien enthält, mit starken Basen, wie Alkali- oder Erdalkalihydroxiden, zu behandeln, um freie Carbonsäure zu neutralisieren, die somit der Reaktion mit Vinyloxiran entzogen wird. Nach dem Neutralisationsschritt wird die wässrige, Acetat enthaltende Phase abgetrennt. Das Verfahren gemäss der obigen Patentschrift liefert in jedem Fall ein salzhaltiges Abwasser, selbst wenn die nach Neutralisation der Essigsäure anfallende, Acetat enthaltende, wässrige Lösung nach Zusatz einer starken Säure aufgearbeitet würde, um die freie Essigsäure wiederzugewinnen.

Es ist somit aus der bisher bekannt gewordenen Literatur über Verfahren zur Herstellung von Vinyloxiran zu entnehmen, dass alle diese Verfahren lediglich Ansätze für eine wirtschaftliche, technische Gewinnung von Vinyloxiran offenbaren, keinesfalls aber eine zufriedenstellende Bewältigung der durch technische Erfordernisse und die Frage der Wirtschaftlichkeit aufgegebenen Problemstellungen zu bieten vermögen.

Demgegenüber wurde nun überraschenderweise gefunden, dass man in technisch einfacher und wirtschaftlich vorteilhafter Weise Vinyloxiran aus Wasserstoffperoxid und Butadien herstellen kann, wenn man

a) eine 10 bis 45 Gew.-% eines wasserlöslichen, sauren Katalysators und 20 bis 50 Gew.-% Wasserstoffperoxid enthaltende wässrige Lösung mit einer 2 bis 5 Kohlenstoffatomen enthaltenden Carbonsäure im Molverhältnis Wasserstoffperoxid zu Carbonsäure wie 0,5 – 30:1 bei Temperaturen von 10 bis 70°C umsetzt,

b) das erhaltene Reaktionsgemisch mit einem inerten, organischen Lösungsmittel im Gegenstrom extrahiert,

c) das im wesentlichen Wasserstoffperoxid und sauren Katalysator enthaltende wässrige Raffinat der Extraktion ganz oder teilweise durch destillative Entfernung von Wasser aufkonzentriert.

d) das aufkonzentrierte Raffinat, sowie den gegebenenfalls nicht aufkonzentrierten Anteil des Raffinats, unter Wiederherstellung der für die Umsetzung mit der Carbonsäure erforderlichen Konzentrationen an Wasserstoffperoxid und wasserlöslichem sauren Katalysator in die Umsetzungsstufe (a) zurückführt, wobei das zur Wiederherstellung der für die Umsetzung mit der Carbonsäure erforderlichen Wasserstoffperoxidkonzentration benötigte Wasserstoffperoxid dem aufzukonzentrierenden Anteil des Raffinats vor oder nach der destillativen Entfernung von Wasser gemäss (c) zugesetzt wird oder zu dem gegebenenfalls nicht aufkonzentrierten Teil des Raffinats zugegeben wird,

e) den im wesentlichen Percarbonsäure und Carbonsäure enthaltenden organischen Extrakt mit Wasser oder einer wässrigen Lösung behandelt und

f) den nunmehr praktisch wasserstoffperoxidfreien, wasserhaltigen, organischen Extrakt einer Azeotropdestillation derart unterwirft, dass der Restgehalt an Wasser im Sumpfprodukt der Azeotropkolonne weniger als 0,5 Gew.-% beträgt,

g) die nunmehr gewonnene, Percarbonsäure und Carbonsäure enthaltende, organische Lösung mit Butadien im Überschuss bei einem Molverhältnis von Butadien zu Percarbonsäure von 1,5 bis 6:1 bei Temperaturen von 0°C bis 80°C und bei einem Druck von 0,8 bis 20 bar umsetzt, und

h) das vinyloxiranhaltige Reaktionsgemisch auf destillativem Wege aufarbeitet, wobei reines Vinyloxiran isoliert wird und das überschüssige Butadien, die Carbonsäure und das inerte organische Lösungsmittel wiedergewonnen und ganz oder teilweise in das Verfahren zurückgeführt werden.

Für das erfindungsgemässe Verfahren geeignete Percarbonsäuren sind solche, die sich von aliphatischen Carbonsäuren mit 2 bis 5 Kohlenstoffatomen ableiten, wie Essigsäure, Propionsäure, n-Buttersäure, Isobuttersäure und Valeriansäure oder Trimethylessigsäure und Dimethylpropionsäure. Besonders geeignet sind Peressigsäure, Perpropionsäure und Perisobuttersäure. Ganz besonders geeignet ist Perpropionsäure.

Bei der Umsetzung gemäss (a) zwischen Wasserstoffperoxid und der Carbonsäure in Gegenwart eines sauren Katalysators bildet sich ein Gleichgewicht zwischen der Carbonsäure und der Percarbonsäure aus, das gemäss folgender Gleichung dargestellt werden kann:

$$R\text{-}\underset{\underset{O}{\|}}{C}\text{-}OH + H_2O_2 \rightleftharpoons R\text{-}\underset{\underset{O}{\|}}{C}\text{-}O\text{-}OH + H_2O$$

Die Carbonsäure setzt sich dabei je nach Konzentration an saurem Katalysator, z.B. Schwefelsäure, und Wasserstoffperoxid in Abhängigkeit des Molverhältnisses von Wasserstoffperoxid zu Carbonsäure zu etwa 30 bis 70% zu Percarbonsäure um.

Im allgemeinen verwendet man neben der 10 bis 45 Gew.-% wasserlöslichen, sauren Katalysator, z.B. Schwefelsäure oder Methansulfonsäure, und 20 bis 50 Gew.-% Wasserstoffperoxid enthaltenden wässrigen Lösung die Carbonsäure in reiner, unverdünnter Form. Man kann aber auch eine wasserhaltige, eine wasserstoffperoxidhaltige oder eine sauren Katalysator enthaltende Carbonsäure einsetzen, wobei man in diesem Fall die Konzentration der wässrigen Lösung entsprechend verändern muss, um das für die Umsetzung erforderliche Mengenverhältnis von Wasserstoffperoxid, saurem Katalysator, Carbonsäure und Wasser einzuhalten. So kann man beispielsweise an Stelle von reiner Carbonsäure auch ein Gemisch aus Carbonsäure und Wasserstoffperoxid, z.B. eine 20 Gew.-% Wasserstoffperoxid enthaltende Carbonsäure einsetzen. Selbstverständlich muss dann entsprechend dem Gehalt an Wasserstoffperoxid in der Carbonsäure der Gehalt an Wasserstoffperoxid in der wässrigen, sauren Katalysator und Wasserstoffperoxid enthaltenden Einsatzlösung angepasst werden, so dass sich aus dem in der Carbonsäure enthaltenden Wasserstoffperoxid und dem der wässrigen Lösung ein Gesamteinsatz von Wasserstoffperoxid ergibt, der einem Gehalt von 20 bis 50 Gew.-% Wasserstoffperoxid in der wässrigen Lösung entspricht. Beispielsweise kann in einem solchen Fall, wo zu Percarbonsäure umzusetzende Carbonsäure bereits Wasserstoffperoxid enthält, in der wässrigen Lösung selbst der Wasserstoffperoxid-Gehalt weniger als 20 Gew.-%, z.B. 12 bis 19 Gew.-% betragen. Innerhalb der angegebenen Konzentrationsverhältnisse von Katalysator und Wasserstoffperoxid kann man alle denkbaren Mischungsverhältnisse anwenden.

Vorzugsweise setzt man eine 20 bis 43, besonders bevorzugt 23 bis 38 Gew.-% sauren Katalysator und 22 bis 35 Gew.-% Wasserstoffperoxid enthaltende wässrige Lösung um.

Als wasserlöslicher saurer Katalysator wird vorteilhafterweise Schwefelsäure benutzt. Es können auch andere wasserlösliche Säuren verwendet werden, z.B. Sulfonsäuren wie Methan-, Äthan-, Propan-, Butan-, Isobutansulfonsäure, Benzolsulfonsäure, Toluolsulfonsäure, Trifluormethan-, 1-Fluoräthan-, Perfluoräthan-, Perfluorpropan- oder Perfluorbutansulfonsäure; Phosphorsäure, Phosphonsäuren, wie Methan- oder Äthanphosphonsäure, Phosphinsäuren, oder saure Salze wie Natriumhydrogensulfat oder Kaliumhydrogensulfat. Man kann auch Gemische wasserlöslicher Säuren verwenden. Als Wasserstoffperoxid wird zur Bereitung der wässrigen Lösung handelsübliches Wasserstoffperoxid, z.B. 30 bis 90 gew.-%iges $H_2O_2$ verwendet. Selbstverständlich ist auch Wasserstoffperoxid geeignet, das als Nebenprodukt anderer chemischer Verfahren oder als Rückführstrom anfällt.

Die Reaktionstemperatur liegt im allgemeinen bei etwa 10 bis 70°C. Zweckmässigerweise arbeitet man bei 20–60°C. Besonders vorteilhaft sind für die Umsetzung Temperaturen unterhalb 43°C, z.B. Temperaturen von 25–43°C.

Im allgemeinen führt man die Umsetzung bis zur Gleichgewichtseinstellung zwischen der Carbonsäure und der Percarbonsäure. Es ist jedoch auch möglich, die Reaktion vor Erreichen des Gleichgewichts abzubrechen und das so erhaltene Reaktionsgemisch der nächsten Verfahrensstufe, d.h. der Extraktion mit dem inerten organischen Lösungsmittel, zuzuführen.

Für die Umsetzung von der Carbonsäure mit Wasserstoffperoxid ist der Druck nicht von Bedeutung, so dass bei Normaldruck, erhöhten Drücken oder auch bei vermindertem Druck gearbeitet werden kann. Im allgemeinen ist es zweckmässig, bei Drücken unterhalb 1,1 bar umzusetzen, z.B. bei Drücken von 0,8 - 1,1 bar.

Die Umsetzung kann in den verschiedensten Reaktionsgefässen durchgeführt werden. Es ist zweckmässig, für ein stationäres Konzentrationsprofil Sorge zu tragen und insbesondere sogenannte tote Zonen, in denen Teile der Reaktionsmischung unverhältnismässig lange verweilen, zu vermeiden. Geeignet sind beispielsweise die üblichen Reaktionsrohre unterschiedlichen Durchmessers und unterschiedlicher Länge, die auch als geschlossener Kreislauf, z.B. als Schlaufenreaktoren, angeordnet sein können, sowie Rührwerkskessel.

Das Reaktionsgemisch aus der Umsetzungsstufe (a) wird nunmehr der Gegenstromextraktion mit dem inerten organischen Lösungsmittel gemäss (b) zugeführt. Diese Gegenstromextraktion kann in einer oder mehreren Extraktionseinheiten durchgeführt werden.

Als Lösungsmittel für die Percarbonsäuren eignen sich alle gegenüber dem Reaktionsgemisch der Umsetzung (a) inerte, mit Wasser nicht mischbare, organische Lösungsmittel. Beispielsweise erwiesen sich als geeignet aromatische Kohlenwasserstoffe, die 6 bis 10 Kohlenstoffatome enthalten, aliphatische oder cycloaliphatische jeweils bis zu 12 Kohlenstoffatome enthaltende Kohlenwasserstoffe, chlorierte Kohlenwasserstoffe, die 1 bis 10 Kohlenstoffatome sowie 1 bis 4 Chloratome enthalten, und Ester von 1 bis 5 C-Atomen enthaltenden Carbonsäuren mit geradkettigen oder verzweigten Alkoholen, in denen 1 bis 8 C-Atome im Molekül vorliegen, sowie Äther, die 2 bis 10 C-Atome enthalten. Beispielsweise seien als geeignete Lösungsmittel genannt: Benzol, Toluol, Xylol, n-Pentan, Isooctan, Cyclohexan, Methylenchlorid, Chloroform, 1,2-Dichloräthan, 1,2-Dichlorpropan, Methylacetat, Äthylacetat, n-Propylacetat, Isopropylacetat, n-Butylacetat, Isoamylacetat, Methylpropionat, Äthylpropionat, Propylpropionat und Butylpropionat, sowie Chlorbenzol und Äther. Es ist aber auch möglich, als Lösungsmittel für die Percarbonsäure Gemische aus den genannten Lösungsmitteln zu verwenden, wobei man dann vorteilhafterweise die Komponenten des Gemisches so wählt, dass sie einen ähnlichen Siedepunkt besitzen. Bevorzugt werden chlorierte Kohlenwasserstoffe, wie Methylenchlorid, Dichloräthan oder Dichlorpropan, aromatische Kohlenwasserstoffe, wie Benzol, oder Äther, wie Diisopropyläther oder Gemische dieser Lösungsmittel verwendet. Ganz besonders bevorzugt wird als Lösungsmittel für das erfindungsgemässe Verfahren Benzol verwendet.

Das Mengenverhältnis von organischem Lösungsmittel zu dem zu extrahierenden, die Percarbonsäure enthaltenden Reaktionsgemisch beträgt im allgemeinen 10 bis 0,1:1. Man kann aber auch grössere Mengen an inertem, organischen Lösungsmittel verwenden. Der jeweils günstigste Bereich des Verhältnisses von gemäss (a) gewonnenem Reaktionsgemisch zu dem für die Extraktion gemäss (b) einzusetzenden organischen Lösungsmittel richtet sich nach der jeweils gewählten Percarbonsäure und den Extraktionseigenschaften des organischen Lösungsmittels und kann vom Fachmann auf einfache Weise bestimmt werden. So ist es beispielsweise für das System Perpropionsäure/Benzol günstig, ein Mengenverhältnis von zu extrahierendem Reaktionsgemisch, das gemäss Stufe (a) gewonnen wurde, zu Benzol von 4 bis 0,3:1 zu wählen. Wird die Extraktion gemäss (b) in mehreren Extraktionseinheiten durchgeführt, so kann die Menge des organischen Lösungsmittels von Einheit zu Einheit innerhalb der angegebenen Grenzen variieren.

Im Extrakt kann der Gehalt an Percarbonsäure durch die Menge des Extraktionsmittels und durch die Zahl der Extraktionsstufen in weiten Grenzen variiert werden. Im allgemeinen arbeitet man so, dass eine etwa 3 bis 40 gew.-%ige Percarbonsäure in dem organischen Lösungsmittel erhalten wird. Bevorzugt gewinnt man einen 5–30 Gew.-% Percarbonsäure enthaltenden, organischen Extrakt. Die Zahl der Extraktionsstufen sollte dementsprechend möglichst hoch sein. Im allgemeinen reicht jedoch eine Extraktionseinheit mit 3 bis 10 theoretischen Extraktionsstufen aus, um die Lösungen mit der gewünschten Konzentration an Percarbonsäuren herzustellen. Selbstverständlich ist es wünschenswert, das Raffinat der Extraktionsstufe (b) möglichst frei von Percarbonsäure und Carbonsäure zu erhalten. Im allgemeinen ist es jedoch ausreichend, wenn im Raffinat nicht mehr als 0,5 Gew.-% Carbonsäure und Percarbonsäure verbleiben.

Für das erfindungsgemässe Verfahren verwendet man eine Percarbonsäure, deren entsprechende Carbonsäure höher als das Vinyloxiran siedet und ein organisches Lösungsmittel, das einen Siedepunkt besitzt, der entweder höher als der Siedepunkt der Percarbonsäure entsprechenden Carbonsäure liegt, oder aber der zwischen dem Siedepunkt von Vinyloxiran und dem der Carbonsäure liegt. Es ist aber auch möglich, das Lösungsmittel so zu wählen, dass sein Siedepunkt unterhalb des Siedepunktes des Vinyloxirans liegt. Als inertes, organisches Lösungsmittel für die Percarbonsäure verwendet man von den bereits aufgeführten Verbindungen bevorzugt ein Lösungsmittel, das bei Normaldruck mindestens 5°C oberhalb des Siedepunktes des Vinyloxirans und mindestens 20°C unterhalb des Siedepunktes der Percarbonsäure entsprechenden Carbonsäure siedet. Daneben erfolgt die Auswahl der Percarbonsäure bzw. der dieser entsprechenden Carbonsäure und des Lösungsmittels für das erfindungsgemässe Verfahren vorteilhafterweise so, dass innerhalb der Kombination Carbonsäure/Lösungsmittel/Vinyloxiran keine ausgeprägten Azeotrope binärer oder ternärer Art auftreten.

Die Temperatur bei der Extraktion kann in weiten Grenzen variiert werden. Im allgemeinen wird bei Temperaturen von 10 bis 70°C gearbeitet. Zweckmässigerweise wählt man die gleiche Temperatur wie bei der Gewinnung der Percarbonsäure gemäss (a), so dass für die Extraktion (b) auch die übrigen für den Reaktionsschritt (a) angeführten Temperaturen in Betracht kommen. Was den Druck anbetrifft, so kann bei Normaldruck, vermindertem Druck oder auch bei erhöhten Drücken gearbeitet werden.

Als Extraktionseinheiten kommen die bekannten Extraktionssysteme in Frage, die eine mehrstufige Gegenstromextraktion ermöglichen.

Geeignet sind z.B. Mischer-Scheider, Siebbodenextraktoren, pulsierte Siebbodenkolonnen oder Sprühkolonnen. Man kann aber auch einstufige oder mehrstufige Zentrifugalextraktoren verwenden.

Der organische Extrakt enthält neben der Percarbonsäure und der Carbonsäure noch geringe Mengen an freiem Wasserstoffperoxid, Wasser und Spuren der als Katalysator verwendeten Säure, z.B. Schwefelsäure. Das Raffinat enthält im wesentlichen das nicht umgesetzte Wasserstoffperoxid und den sauren Katalysator.

Das im wesentlichen Wasser, Wasserstoffperoxid und z.B. Schwefelsäure als sauren Katalysator enthaltende Raffinat der Extraktion wird nun im Verfahrensschritt (c) dadurch wieder für die Umsetzung von der Carbonsäure und Wasserstoffperoxid aufbereitet, dass man es ganz oder teilweise durch Entfernung von Wasser in einer Destillation aufkonzentriert. Die aus dem dieser Aufkonzentrierung zugeführten Raffinatstrom abzudestillierende Wassermenge entspricht im wesentlichen sowohl der Menge an Wasser, die bei der Umsetzung von Wasserstoffperoxid mit der Carbonsäure gemäss (a) entstanden ist als auch der Wassermenge, die mit dem frischen Wasserstoffperoxid, das zur Ergänzung der verbrauchten Mengen benötigt wird, in das Verfahren eingebracht wird. Als Kopfprodukt der Destillation erhält man Wasser, das geringe Mengen an Wasserstoffperoxid, Percarbonsäure und Carbonsäure enthalten kann. Im allgemeinen führt man die Destillation unter vermindertem Druck durch, z.B. bei Drücken von 10 bis 250 Torr, vorzugsweise 40 bis 150 Torr, und bei Temperaturen im Sumpf von 40 bis 120°C, vorzugsweise von 60 bis 85°C. Im allgemeinen ist für die Aufkonzentrierung auch der gesamte, die Extraktion verlassende Raffinatstrom geeignet, wenn die Extraktion in einer einzigen Extraktionseinheit durchgeführt wird. Man kann aber auch bei einer in beispielsweise zwei Extraktionseinheiten erfolgenden Extraktion des gemäss (a) erhaltenen Reaktionsgemisches sowohl das Raffinat aus der ersten als auch das Raffinat aus der zweiten Extraktionsstufe der Aufkonzentrierung zuführen. Wird bei einer in zwei Extraktionseinheiten erfolgenden Extraktion das Raffinat der ersten Einheit in einen grösseren und einen kleineren Teilstrom aufgeteilt, so ist grundsätzlich jeder dieser Anteile für die Aufkonzentrierung geeignet. Vorteilhafterweise unterwirft man bei einer aus zwei Extraktionseinheiten bestehenden Extraktion, bei der das die erste Einheit verlassende Raffinat in einen kleinen und einen grösseren Teilstrom aufgeteilt wird und der kleinere Anteil in die zweite Einheit eingegeben wird, das Raffinat aus der zweiten Extraktionseinheit der Destillation zur Aufkonzentrierung.

Das frische Wasserstoffperoxid zur Ergänzung der verbrauchten Mengen kann in beliebiger Konzentration zugegeben werden. Zweckmässigerweise verwendet man handelsübliches, z.B. 30 bis 90 gew.-%iges wässriges Wasserstoffperoxid, das mit den üblichen Stabilisatoren versetzt werden kann. Beispielsweise kommen Stabilisatoren in Betracht, wie die in Gmelins «Handbuch der anorganischen Chemie», 8. Auflage, Sauerstoff-Band, Lieferung 7, 1966, auf Seite 2274 und Seite 2275 aufgeführten.

Man kann das frische Wasserstoffperoxid vor Eintritt in die Destillationseinheit mit dem aufzukonzentrierenden Raffinat aus der Extraktion gemäss der Verfahrensstufe (b) mischen; man kann die beiden Mengenströme auch getrennt in die Destillationseinheit führen. Ebenso ist es möglich, das frische Wasserstoffperoxid dem Raffinat nach erfolgter Aufkonzentrierung zuzusetzen. Man kann auch einen Teil des frischen Wasserstoffperoxids dem Raffinat vor der Aufkonzentrierung zumischen und die restliche Menge des frischen Wasserstoffperoxids nach erfolgter Aufkonzentrierung dem Raffinat zusetzen.

Man kann aber auch das frische Wasserstoffperoxid direkt in die Umsetzung gemäss (a) eingeben, oder aber auch dem nicht zur Aufkonzentrierung gelangenden Raffinatanteil der Extraktion zumischen. Als Destillationseinheit verwendet man zweckmässigerweise eine mit Kondensator und einer Verdampfereinheit versehene Kolonne.

Für die Destillation kommen die bekannten Bodenkolonnen oder Füllkörperkolonnen in Frage. Die Zahl der Destillationsstufen wird so gewählt, dass das Kopfprodukt möglichst wenig Wasserstoffperoxid enthält. Erwünscht ist es, weniger als 0,1 Gew.-% Wasserstoffperoxid im Kondensat zu erhalten. Als Verdampfereinheit eignen sich grundsätzlich die bekannten Verdampfer. Geeignet sind beispielsweise solche Verdampfereinheiten, in denen die Verweilzeit des Produktes weniger als 20 Minuten, vorzugsweise weniger als 10 Minuten, beträgt. Besonders geeignet sind Fallstrom- oder Dünnschichtverdampfer. Als Materialien für die Destillationseinheit eignen sich hochlegierte, rostfreie Edelstähle, die neben Eisen im wesentlichen noch Chrom und Nickel enthalten, wie beispielsweise ein Werkstoff mit der DIN-Bezeichnung 1.4571, der neben Eisen 17,5 Gew.-% Chrom, 11,5 Gew.-% Nickel, 2,25 Gew.-% Molybdän, sowie bis zu 2 Gew.-% Mangan, bis zu 1 Gew.-% Silicium, bis zu 0,1 Gew.-% Kohlenstoff und geringe Mengen Titan enthält, oder ein Werkstoff, der neben Eisen 25 Gew.-% Chrom, 25 Gew.-% Nickel, 2,25 Gew.-% Molybdän und bis zu 2 Gew.-% Mangan, bis zu 1 Gew.-% Silicium, bis zu 0,06 Gew.-% Kohlenstoff sowie geringe Mengen Titan enthält und nach DIN mit der Nummer 1.4577 bezeichnet wird. Besonders geeignet sind als Werkstoff für die Destillationseinheit, insbesondere für den Verdampfer, Zirkon, zirkonhaltige Werkstoffe und Zirkonlegierungen.

Das Sumpfprodukt dieser Destillationseinheit wird – gegebenenfalls unter Wiederherstellung der für die Umsetzung mit der Carbonsäure erforderlichen Konzentrationen an Wasserstoffperoxid und Katalysator – in die Umsetzungsstufe (a) zurückgeleitet. Aufgrund dieser Massnahme, das Raffinat der Extraktion in die Umsetzungsstufe gemäss (a) zurückzuführen, wobei es zuvor ganz oder teilweise die Aufkonzentrierung gemäss (c) durchlaufen hat, erhält man einen im wesentlichen die Verfahrensstufen (a), (b), (c), und (d) umfassenden Kreislauf von Wasserstoffperoxid und Katalysator. Dabei kann es zweckmässig sein, einen Teil, z.B. 0,1 bis 6 Gew.-% des Kreislaufstromes von Zeit zu Zeit oder auch kontinuierlich als Seitenstrom aus dem Verfahren zu entnehmen. Vorteilhafterweise entnimmt man diesen Seitenstrom an einer Stelle des Verfahrens, wo der Kreislaufstrom eine möglichst geringe Konzentration an Wasserstoffperoxid und saurem Katalysator sowie gegebenenfalls an Percarbonsäure und Carbonsäure besitzt. Ganz besonders eignet sich für diese Seitenstromentnahme das Raffinat der Extraktion vor erfolgter Zugabe von Frisch-Wasserstoffperoxid und vor erfolgter Aufkonzentrierung gemäss (c). Dieser Seitenstrom, der als Teil des Kreislaufstromes eine wässrige, im wesentlichen Wasserstoffperoxid und sauren Katalysator enthaltende Lösung darstellt, kann entweder verworfen werden oder zur Aufarbeitung in eine Regenerationsstufe geführt werden. Eine Regeneration dieses Anteils des Kreislaufstromes kann beispielsweise so erfolgen, dass man das darin enthaltene Wasserstoffperoxid im Vakuum mit Wasserdampf abdestilliert, wobei als Destillationsrückstand eine wässrige Lösung des sauren Katalysators anfällt. Die als Destillat anfallende wässrige, Wasserstoffperoxid enthaltende Lösung kann, gegebenenfalls nach Aufkonzentrieren, in den Prozess zurückgeleitet werden. Die wässrige Lösung des sauren Katalysators kann nach Reinigung, beispielsweise durch Destillation, ebenfalls in den Prozess zurückgeführt werden. Durch diesen Kreislaufaustausch wird dem Verfahren ein entsprechender Teil des Katalysators, z.B. der Schwefelsäure, entzogen, der somit im Verfahren ergänzt werden muss.

Der im wesentlichen die Percarbonsäure und die Carbonsäure enthaltende organische Extrakt, der gemäss Verfahrensstufe (b) erhalten wird, wird im Verfahrensschritt (e) mit Wasser oder einer wässrigen Lösung behandelt. Im allgemeinen verfährt man dabei so, dass man den organischen Percarbonsäure-Extrakt in einer der dafür üblichen Vorrichtungen mit Wasser wäscht.

Es ist zweckmässig, diese Wäsche als Extraktion, z.B. als mehrstufige Gegenstromextraktion, mit Wasser, beispielsweise in einer dreistufigen Extraktionseinheit, durchzuführen. An Stelle der Gegenstromextraktion kann selbstverständlich auch eine Extraktion im Gleichstrom bzw. Kreuzstrom angewendet werden. Man kann beim Arbeiten mit mehreren Extraktionsstufen die Extraktion auch teilweise im Gleichstrom und teilweise im Gegenstrom, durchführen.

Man verwendet zweckmässigerweise 0,1 bis 10 Volumen-% an Wasser oder wässriger Lösung, bezogen auf den organischen Extrakt. Vorzugsweise verwendet man 0,5 bis 7 Volumen-% Wasser. Anstelle von reinem Wasser kann man auch eine wässrige Lösung verwenden, die im wesentlichen frei von Wasserstoffperoxid und von Mineralsäure ist. Zweckmässig ist es, eine wässrige Phase zu verwenden, die im Verfahren anfällt. Geeignet ist beispielsweise das bei der Durchführung des Verfahrensschrittes (d) gewonnene Destillat. Die wässrige Phase der Wasserbehandlung kann man in die Extraktion gemäss (b) zurückleiten, um die darin befindlichen Anteile an Percarbonsäure, Carbonsäure und Wasserstoffperoxid für das Verfahren zu erhalten.

Als Vorrichtungen für die Wasserbehandlung gemäss Verfahrensstufe (e) sind die bekannten Extraktionssysteme geeignet, z.B. Mischer-Scheider, Siebbodenextraktoren, pulsierte Siebbodenkolonnen oder Extraktionszentrifugen.

Nach erfolgter Behandlung der organischen Lösung der Percarbonsäure mit Wasser oder einer wässrigen Lösung gemäss (e) erhält man eine im wesentlichen Wasserstoffperoxid- und Schwefelsäure-freie, organische Lösung der Percarbonsäure, die nunmehr der Azeotropdestillation gemäss Verfahrensstufe (f) unterworfen wird, wobei in der organischen Lösung enthaltenes Wasser entfernt wird. Der Gehalt an Wasser der zur Trennung gelangenden organischen Lösung beträgt im allgemeinen 0,5 bis 7 Gew.-%. Dieser Wassergehalt ist im wesentlichen sowohl von der Natur des jeweils verwendeten organischen Lösungsmittels als auch von der gewählten Konzentration an Percarbonsäure im gemäss (b) erhaltenen Extraktes abhängig.

Das zur Entfernung von Wasser aus der organischen Lösung der Percarbonsäure durch Azeotropdestillation verwendete Schleppmittel ist im allgemeinen das inerte organische Lösungsmittel. Es ist aber auch möglich, ein anderes geeignetes Schleppmittel der organischen Lösung der Percarbonsäure vor Eintritt in die Verfahrensstufe (f) oder direkt in die Destillationseinheit gemäss (f) zuzusetzen und mit diesem die Azeotropdestillation durchzuführen.

In der Verfahrensstufe (f) wird im allgemeinen die Destillatmenge so gewählt, dass der Restgehalt an Wasser im Sumpfprodukt der Azeotropkolonne weniger als 0,5 Gew.-%, vorzugsweise weniger als 0,2 Gew.-%, z.B. 0,05-0,2 Gew.-%, beträgt. Es ist aber auch möglich, den Wassergehalt auf einen vernachlässigbar kleinen Wert zu reduzieren. Das nach Kondensation der Kopfdämpfe der Azeotropkolonne sich als organische Phase abscheidende Schleppmittel führt man der Kolonne als Rücklauf zu. Die nach Kondensation der Kopfdämpfe erhaltene wässrige Phase, die im allgemeinen geringe Mengen an Percarbonsäure, Carbonsäure sowie Wasserstoffperoxid enthält, wird dem Verfahren an geeigneter Stelle, beispielsweise der Extraktion gemäss (e) oder (b) wieder zugeführt; sie kann aber auch aus dem Verfahren ausgeschleust werden.

Die Azeotropdestillation (f) kann bei normalem oder vermindertem Druck, beispielsweise bei 200 bis 400 Torr, durchgeführt werden. Die Sumpftemperatur liegt beispielsweise bei 30 bis 80°C. Im allgemeinen ist eine Sumpftemperatur von unterhalb 70°C ausreichend.

Für die Azeotropdestillation eignen sich die üblichen Kolonnen, beispielsweise die bekannten Boden- oder Füllkörperkolonnen. Als Verdampfer kann man die üblichen Vorrichtungen verwenden. Bevorzugt geeignet sind Fallstrom- oder Dünnschichtverdampfer.

Die nunmehr als Sumpf der Azeotropdestillation erhaltene, organische Lösung einer im wesentlichen wasser- und wasserstoffperoxidfreien Percarbonsäure wird im Verfahrensschritt (g) mit einem Überschuss an Butandien im Molverhältnis von Butadien zu Percarbonsäure von 1,5 bis 6:1 bei Temperaturen von 0°C bis 80°C und bei einem Druck von 0,8 bis 20 bar umgesetzt.

Man kann aber auch bei einem Druck von 1 bis 10 bar arbeiten. Ein geeigneter Druckbereich sind beispielsweise Drücke von 1,2 bis 8 bar. Bevorzugt arbeitet man bei einem Druck von 1,5 bis 5 bar.

Die Reaktionstemperatur wird vorzugsweise bei 10 bis 70°C gehalten. Ganz besonders bevorzugt führt man die Umsetzung von Butadien mit der organischen Lösung der Percarbonsäure bei Temperaturen von 20 bis 60°C durch. Neben der Arbeitsweise unter isothermen Bedingungen, d.h. Einhaltung einer einheitlichen Temperatur im gesamten Reaktionsgemisch, kann man die Umsetzung auch unter Ausbildung eines sogenannten Temperaturgradienten durchführen, der im allgemeinen mit fortschreitender Reaktion zunimmt. Man kann aber auch die Reaktion so führen, dass sich mit dem Fortschreiten der Reaktion ein Gradient fallender Temperatur ausbildet.

Der Druck bei der Durchführung des Verfahrensschrittes (g) wird zweckmässigerweise so gewählt, dass die Reaktionsmischung im wesentlichen in flüssiger Phase vorliegt. Bei einem Molverhältnis von Butadien zu Percarbonsäure von beispielsweise 2,5:1 und bei 40 bis 50°C Reaktionstemperatur beträgt der Druck beispielsweise 2 bis 3,5 bar.

Das Molverhältnis von Butadien zu Percarbonsäure beträgt vorzugsweise 1,5 bis 4:1. Ganz besonders vorteilhaft is es, ein Molverhältnis von 2,0 bis 3,0 Mol Butadien je Mol Percarbonsäure anzuwenden.

Zur Durchführung der Reaktion können die für Umsetzungen dieser Art üblichen Vorrichtungen verwendet werden, wie Rührwerkskessel, Röhrenreaktoren, Schlaufenreaktoren oder Schleifenreaktoren. Im allgemeinen verwendet man eine Vorrichtung, die sich wie eine Kaskade von mindestens zwei ideal durchmischten Kesseln verhält. Besonders vorteilhaft ist es, ein Reaktionssystem zu verwenden, das sich wie eine Kaskade von 4 bis 50, vorzugsweise 10 bis 30, ideal durchmischten Kesseln verhält. Bei der praktischen Durchführung der Umsetzung verwendet man z.B. eine Folge von mehreren Rührwerkskesseln, beispielsweise eine Kaskade von 3 bis 6 Kesselreaktoren.

Für die Umsetzung gemäss Verfahrensschritt (g) wird im allgemeinen technisches Butadien verwendet. Es kann die beim technischen Gebrauch üblichen Beimengungen, insbesondere n-Butene enthalten.

Das Butadien kann auf verschiedene Art in die Reaktionseinheit eingebracht werden. Man kann das Butadien in flüssiger oder gasförmiger Form einsetzen. Man kann das Butadien auch gemeinsam mit der Percarbonsäurelösung in die Reaktoreinheit leiten. Es können auch beide Einsatzmaterialien getrennt voneinander in den Reaktor eingeführt werden. Weiterhin ist es möglich, das Butadien und die Percarbonsäurelösung an verschiedenen Stellen in die Reaktoreinheit zu leiten. Bei Verwendung mehrerer in Kaskade geschalteter Reaktoren kann es zweckmässig sein, das gesamte Butadien in den ersten Reaktor einzubringen. Man kann das Butadien aber auch auf die verschiedenen Reaktoren aufteilen.

Die erhebliche Reaktionswärme wird durch innen- und aussenliegende Kühler abgeführt. Zur Ableitung der Reaktionswärme kann die Umsetzung auch unter Rückfluss, d.h. in Siedereaktoren, durchgeführt werden. Die Reaktion wird zweckmässigerweise unter möglichst vollständiger Umsetzung der Percarbonsäure vorgenommen. Im allgemeinen setzt man mehr als 98% der Percarbonsäure um. Zweckmässig ist es, mehr als 99% Percarbonsäure umzusetzen. Die Umsetzung kann mit einer besonders hohen Selektivität durchgeführt werden, wenn man sie teilweise in einem von turbulenter Strömung durchflossenen Reaktionsrohr, das beispielsweise an die Folge von Rührwerkskesseln angeschlossen ist, durchführt. Besonders günstig ist es, ein Reaktionsrohr, das mit Einbauten, die eine Rückvermischung weitgehend verhindern, z.B. gelochten Querböden, ausgestattet ist, zu verwenden. Beispielsweise führt man die Umsetzung zunächst in einigen, z.B. 1 bis 3, in Serie geschalteten, gerührten Reaktionseinheiten durch und leitet das Reaktionsgemisch sodann zur Vervollständigung der Umsetzung in ein Reaktionsrohr. Das Reaktionsrohr kann unter adiabatischen Bedingungen betrieben werden; man kann aber auch kühlen, beispielsweise durch Aussenkühlung, oder man kann zwischen einzelnen Rohrabschnitten Kühler anbringen. Die Dimensionierung eines geeigneten Reaktionsrohres hängt von dem beabsichtigten Durchsatz ab. Wesentlich ist es, dass die Strömungsgeschwindigkeit in dem Reaktionsrohr so hoch ist, dass die Rückvermischung der Reaktionskomponenten im wesentlichen ausgeschlossen wird. Der Durchmesser des Reaktionsrohrs kann 0,01 bis 10 Meter betragen bei einer Länge von 1 bis 200 Metern. Man kann auch mehrere Rohre parallel betreiben. Beispielsweise kann man auch Rohrbündel einsetzen. Falls ein Reaktionsrohr mit gelochten Querböden benutzt wird, befinden sich die Querböden im allgemeinen in einem Abstand von 0,1 bis 5 m voneinander.

Bei der erfindungsgemässen Durchführung der Umsetzung zwischen Butadien und der Percarbonsäure (Schritt g) gelingt es, Vinyloxiran-Ausbeuten von über 95%, bezogen auf eingesetzte Percarbonsäure, zu erzielen. Die Menge der Nebenprodukte, z.B. das Buten(1)-diol-(3,4) und dessen Mono- und Diester mit der der Percarbonsäure entsprechenden Carbonsäure, beträgt dabei weniger als 2%, bezogen auf gebildetes Vinyloxiran. Die Menge an polymerisiertem Butadien beträgt, bezogen auf die gesamte, in die Reaktionsstufe gemäss (g) eingesetzte Butadienmenge, weniger als 1 Gew.-%.

Die Aufarbeitung des Reaktionsgemisches erfolgt auf destillativem Wege, wobei reines Vinyloxiran gewonnen wird und überschüssiges Butadien, die Carbonsäure und das organische Lösungsmittel in einem solchen Reinheitsgrad isoliert wird, dass eine Rückführung, die gegebenenfalls auch nur teilweise erfolgen kann, in das Verfahren möglich ist. Es ist zweckmässig, Vinyloxiran und die Carbonsäure sehr schnell voneinander zu trennen. Dazu dient beispielsweise eine Destillationskolonne, in der man zunächst Vinyloxiran und Butadien, gegebenenfalls zusammen mit niedriger siedenden Bestandteilen und einem Teil des Lösungsmittels über Kopf abnimmt und das restliche Lösungsmittel und die Carbonsäure als Sumpfprodukt erhält. Das Kopfprodukt wird nach einer ebenfalls auf destillativem Wege vorgenommenen Abtrennung des Butadiens in einer weiteren Destillationskolonne zur Gewinnung von reinem Vinyloxiran aufgearbeitet. Aus den Sümpfen dieser beiden Destillationskolonnen gewinnt man das organische Lösungsmittel und die Carbonsäure wieder. Destillationsrückstand der Carbonsäuredestillation ist die bereits erwähnte geringe Menge an höhersiedenden Nebenprodukten. Das organische Lösungsmittel kann grundsätzlich quantitativ wiedergewonnen werden.

In vielen Fällen kann es von Vorteil sein, das die Verfahrensstufe gemäss (g) verlassende Reaktionsgemisch vor Eintritt in die erste Destillationskolonne mit einem Stabilisator oder einer Lösung eines Stabilisators zu versetzen. Man kann aber auch ohne Anwendung eines Stabilisators das Reaktionsgemisch destillativ aufarbeiten. Als Stabilisatoren kommen alle Verbindungen in Frage, die Sauerstoff oder Spuren peroxidischer Verbindungen zu binden in der Lage sind. Diese Verbindungen können auch Stickstoff und/oder Schwefel enthalten. Beispielsweise seien erwähnt Hydrochinon, 4-tert.-Butylbrenzkatechin, 2,6-Di-ter.-Butyl-4-methyl-phenol, Phenothiazin, N,N-Dimethylanilin, Tetramethyl-hydrochinon, N-Nitroso-diphenylamin, Pyrogallol, 2-Methyl-benzothiazol, 6-Methoxy-2-amino-benzothiazol, 2,3-Dihydroxychinoxalin und die Additionsverbindung aus Diisobutylen und Stickstoffoxid.

Eine Ausführungsform des erfindungsgemässen Verfahrens sei anhand von Abbildung 1 erläutert.

In die erste Reaktionsstufe (1) werden gleichzeitig über (2) eine 30 bis 40 Gew.-% Schwefelsäure und 25 bis 35 Gew.-% Wasserstoffperoxid enthaltende wässrige Lösung und über (3) Propionsäure im Molverhältnis von Wasserstoffperoxid zu Propionsäure wie 0,9 bis 1,2:1 bei einer Temperatur von 25 bis 45°C gegeben. Die Verweilzeit im Reaktionssystem (1) beträgt 10 bis 30 Minuten. Das Reaktionsgemisch, das das Reaktionssystem (1) über (4) verlässt, enthält ca. 26 bis 32 Gew.-% Perpropionsäure, 12 bis 17 Gew.-% Propionsäure, 17 bis 21 Gew.-% Schwefelsäure, 5 bis 8 Gew.-% Wasserstoffperoxid und 2 bis 5 Gew.-% Carosche Säure. Es gelangt in ein Extraktionssystem (5), das aus einer pulsierten Siebbodenkolonne mit 60 bis 90 Siebböden besteht und das mit Benzol mit einem Propionsäuregehalt von weniger als 0,5% über (6) beschickt wird. Das Raffinat dieser Extraktion, das über (7) aus dem Extraktionssystem (5) abgeführt wird, enthält das im Reaktionssystem (1) nicht umgesetzte Wasserstoffperoxid und die Schwefelsäure. Es wird gemeinsam mit ca. 50%igem handelsüblichen wässrigen Wasserstoffperoxid, das über (9) zugeführt wird, in die aus Verdampfer und Kolonne bestehende Destillationseinheit (8) geleitet, in der bei 40–120 Torr und einer Sumpftemperatur von 60 bis 80°C so viel Wasser über Kopf entnommen wird, dass als Sumpfprodukt eine 30 bis 40 Gew.-% Schwefelsäure und 25 bis 35 Gew.-%

Wasserstoffperoxid enthaltende wässrige Lösung erhalten wird, die über (2) wieder in das Reaktionssystem (1) zurückgeführt wird. Das in der Destillationseinheit (8) über Kopf genommene Wasser wird aus dem Verfahren über (10) entnommen. Die Wassermenge, die als Destillat erhalten wird, entspricht im wesentlichen der im Einsatzwasserstoffperoxid enthaltenen und in der Verfahrensstufe gemäss (a), also im Reaktionssystem (1) gebildeten Wassermenge. Als Verdampfereinheit der Destillationskolonne (8), verwendet man einen Fallstromverdampfer. Der benzolische Perpropionsäureextrakt aus dem Extraktionssystem (5) wird über (11) in das aus 3 Mischer-Scheidern bestehende Extraktionssystem (12) geleitet, wo der Extrakt mit Wasser, das über (13) zugeführt wird, im Gegenstrom extrahiert wird. Die Wassermenge beträgt 3 bis 6 Volumenprozent der benzolischen Lösung. Die wässrige Phase dieser Extraktionseinheit (12) wird über (14) in die Extraktionseinheit (5) zurückgeführt.

Die mit Wasser behandelte benzolische Perpropionsäurelösung gelangt über (15) in die Destillationseinheit (16), wo eine azeotrope Entwässerung durchgeführt wird. Der Druck innerhalb des Destillationssystems (16) beträgt 100 bis 300 Torr. Die Sumpftemperatur liegt bei 50 bis 75°C. Der Wassergehalt der aus dem Sumpf dieser Kolonne abfliessenden benzolischen Perpropionsäure beträgt weniger als 0,3 Gew.-%. Die als Sumpf der Azeotropdestillation erhaltene, im wesentlichen wasser- und wasserstoffperoxidfreie benzolische Perpropionsäure wird über (17) in das Reaktionssystem (18) geleitet, wo die Umsetzung mit Butadien im Molverhältnis von Butadien zu Perpropionsäure wie 2 bis 4:1 erfolgt. Das Butadien gelangt über (19), (20) und (22) in das Reaktionssystem (18). Der Druck in (18) beträgt 3–5 bar. Das Reaktionssystem (18) besteht aus 2 in Serie geschalteten Schlaufenreaktoren mit einem nachgeschalteten Verweilzeitrohr von 10 bis 80 m Länge. Die Temperatur in den beiden Schlaufenreaktoren, in denen die Durchmischung der Reaktionsteilnehmer mittels Umlaufpumpe vorgenommen wird, liegt bei 20 bis 50°C. Die Perpropionsäure wird dabei zu 80 bis 95% umgesetzt. Die weitere Reaktion der Perpropionsäure bis zu einem Umsatz von 99,8% erfolgt in dem nachgeschalteten Verweilzeitrohr, das ohne Kühlung betrieben wird. Das erhaltene Reaktionsgemisch wird über (23) in ein Entspannungsgefäss (21) geführt und dort entspannt. Die dabei erhältliche Gasphase enthält im wesentlichen Butadien, das über (22) in die Umsetzung mit Perpropionsäure zurückgeführt wird. Aus der flüssigen Phase, die über (24) in die Destillationseinheit (25) gelangt, wird zunächst Vinyloxiran zusammen mit Butadien und mit einem Teil des Benzols durch Destillation abgetrennt. Der Butadien, Vinyloxiran und Benzol enthaltende Strom wird über (26) der Destillationseinheit (27) zugeführt, wo die weitere Auftrennung der Komponenten erfolgt und reines Vinyloxiran gewonnen wird, das das Verfahren über (28) verlässt. Butadien wird über (20) wieder in das Reaktionssystem (18) zurückgeführt. Die Sümpfe der Kolonne (25) und (27) werden über (29) und (30) einer weiteren Destillationseinheit (31) zugeführt, wo Benzol als Kopfprodukt wiedergewonnen und über (6) in das Extraktionssystem (5) zurückgeführt wird. Der im wesentlichen aus Propionsäure bestehende Sumpf der Benzol-Rückgewinnungskolonne (31) wird über (32) der Destillationseinheit (33) zugeführt, in der als Kopfprodukt Propionsäure abdestilliert wird, welche über (3) in das Reaktionssystem (1) zurückgeführt wird. Die höher als Propionsäure siedenden Produkte werden als Sumpfprodukt der Destillation (33) erhalten und über (34) ausgeschleust.

Nach dem erfindungsgemässen Verfahren kann Vinyloxiran in Ausbeuten von mindestens 90%, bezogen auf eingesetztes Wasserstoffperoxid, und mindestens 95%, bezogen auf eingesetztes Butadien, hergestellt werden.

Beispiel 1 (s. auch Fig. 2)

Durch das aus einem mit Füllkörpern versehenen, beheizbaren Verweilzeitrohr von 55 cm Länge und einem Durchmesser von 5 cm bestehenden Reaktionssystem (1) werden bei kontinuierlichem Betrieb in der Stunde 1034 g eines Gemisches aus 415 g (= 5,6 Mol) Propionsäure und 619 g einer wässrigen Lösung, die 37,8 Gew.-% Schwefelsäure und 30,8 Gew.-% Wasserstoffperoxid (= 5,6 Mol $H_2O_2$) enthält, geleitet.

In der Wasserstoffperoxid und Schwefelsäure enthaltenden wässrigen Lösung liegt ein Teil dieser Komponenten als Carosche Säure vor. Dieser Teil beträgt 5,8 Gew.-%, bezogen auf die Gesamtmenge der wässrigen Lösung, für die sich somit folgende Zusammensetzung ergibt:

32,8 Gew.-% freie Schwefelsäure, 29,1 Gew.-% freies Wasserstoffperoxid und 5,8 Gew.-% Carosche Säure. Das Molverhältnis von Wasserstoffperoxid zu Propionsäure beträgt in dem in das Reaktionssystem (1) gelangenden Gemisch 1:1, wobei das in der Caroschen Säure gebundene Wasserstoffperoxid als freies $H_2O_2$ gerechnet wird.

Innerhalb des Reaktionssystems (1) wird das nunmehr aus Propionsäure, Schwefelsäure, Wasserstoffperoxid, Wasser und Caroscher Säure bestehende Gemisch etwa 18 Minuten lang auf 40°C erwärmt, wobei sich die Propionsäure zu 59% zu Perpropionsäure umsetzt. Nach Durchlaufen des Verweilzeitrohres wird der im Mittel 28,8 Gew.-% Perpropionsäure, 16,5 Gew.-% Propionsäure, 19,6 Gew.-% Schwefelsäure, 3,47 Gew.-% Carosche Säure, 6,54 Gew.-% Wasserstoffperoxid und 25,1 Wasser enthaltende Produktstrom (1034 g pro Stunde) auf Raumtemperatur abgekühlt und in einen Gasabscheider geleitet, wo pro Stunde 166 ml eines aus 87% Sauerstoff und 13% Kohlendioxid bestehenden Gases entweichen. Dann wird das entgaste Gemisch, nachdem man es mit dem aus der Extraktionseinheit (12) ablaufenden Gemisch der beiden wässrigen Phasen vereinigt hat, dem Extraktionssystem (5) zugeführt. Der Extraktionsvorgang wird bei einer Temperatur von 32°C vorgenommen. Als Extraktionssystem verwendet man eine pulsierte Siebbodenkolonne, die mit 80 Siebböden versehen ist, eine Länge von 4 m und einen Durchmesser von 25 mm besitzt, sowie am oberen und unteren Ende mit je einem Abscheidegefäss, in denen die Phasentrennung erfolgt, ausgestattet ist. Am oberen Ende der Kolonne (5) wird der nach der Entgasung erhaltene und mit dem über Leitung (14) zugeführten Gemisch der wässrigen Phasen des Extraktionssystems (12) vereinigte Produktstrom aufgegeben, der als schwere Phase die Kolonne von oben nach unten durchströmt, während am unteren Ende das als Extraktionsmittel dienende Benzol, das 0,09 Gew.-% Propionsäure sowie Spuren von Wasser enthält, mit einer Menge von 1092 ml pro Stunde (= 961 g/h) in die Kolonne eingegeben wird. Aus dem oberen Scheidegefäss zieht man in der Stunde 1585 ml einer benzolischen Lösung von Perpropionsäure (= 1490 g/h) ab, die neben 21,4 Gew.-% Perpropionsäure noch 12,6 Gew.-% Propionsäure, 0,97 Gew.-% Wasser, 0,51 Gew.-% Wasserstoffperoxid sowie Spuren von Schwefelsäure enthält.

Das Raffinat der Extraktion sammelt sich als schwere Phase in dem unteren Scheidegefäss und wird von dort über Leitung (7) kontinuierlich entfernt. Dieses Raffinat, das mit einer Menge von etwa 587 g pro Stunde anfällt, enthält im Mittel 34,58 Gew.-% Schwefelsäure, 11,16 Gew.-% Wasserstoffperoxid, 6,12 Gew.-% Carosche Säure, sowie 0,1 Gew.-% Propionsäure und 0,06 Gew.-% Perpropionsäure. Man bereitet dieses Raffinat für die Umsetzung mit Propionsäure wieder auf, indem man es pro Stunde über (9) mit 195,6 ml einer 50%igen wässrigen Lösung von Wasserstoffperoxid (= 117 g = 3,44 Mol $H_2O_2$) versetzt und das so erhaltene Gemisch durch Abdestillieren von 202 g Wasser wieder aufkonzentriert. Dieser Vorgang der Aufkonzentrierung erfolgt in der Destillationseinheit (8), die aus einer mit Glockenböden versehenen Kolonne (Länge = 1 m, Durchmesser = 50 mm), einem Kondensator, einer Vorrichtung, die es gestattet, das Rücklaufverhältnis zu variieren, sowie einem Fallfilmverdampfer, der, mit den Dämpfen einer siedenden Flüssigkeit beheizbar ist, besteht und bei einem Druck von 50 Torr betrieben wird. Das aus dem Raffinat der Extraktion (5) und der wässrigen Lösung von Wasserstoffperoxid bestehende Gemisch wird in den unteren Teil der Kolonne (8) eingegeben. Bei einer Sumpftemperatur von 68–70°C, einer Temperatur am Kopf der Kolonne von 32°C, und einem Rücklaufverhältnis von 0,7 (Rücklauf/Entnahme) destillieren in der Stunde 202 ml Wasser über. Dieses Destillat enthält Spuren Wasserstoffperoxid sowie 0,2 Gew.-% Perpropionsäure und 0,3 Gew.-% Propionsäure. Aus dem Sumpf der Kolonne werden über Leitung (2) pro Stunde 619 g einer wässrigen Lösung abgezogen, die wiederum 32,8 Gew.-% Schwefelsäure, 29,1 Gew.-% Wasserstoffperoxid und 5,8 Gew.-% Carosche Säure enthält. Dieses Gemisch wird, nachdem es auf Raumtemperatur abgekühlt wurde, dem Reaktionssystem (1) wieder zugeführt.

Aus dem sich so ausbildenden, das Reaktions- und Extraktionssystem (1) bzw. (5), sowie die Destillationseinheit (8) umfassenden Kreislaufstrom von Wasserstoffperoxid und Schwefelsäure werden in der Stunde etwa 4,5 g als Raffinat der Extraktion (5) ausgeschleust. Den somit im Kreislauf entstandenen Verlust an Schwefelsäure ergänzt man, indem man pro Stunde die gleiche Menge eines die Zusammensetzung des Raffinates der Extraktion (5) besitzendes Gemisch kontinuierlich vor der Destillationseinheit (8) in den Kreislauf eingibt.

Der durch diesen Kreislaufaustausch resultierende Verlust an Wasserstoffperoxid beträgt 0,5%, bezogen auf das vor der Destillationseinheit (8) eingesetzte Frisch-Wasserstoffperoxid.

Die dem Extraktionssystem (5) als leichte Phase entnommene benzolische Lösung von Perpropionsäure wird über Leitung (11) dem Extraktionssystem (12), das als dreistufige übereinander angeordnete Mischer-Scheider-Batterie bestehend aus jeweils einer Mischpumpe mit nachfolgendem Scheidegefäss von etwa 2 Liter Inhalt ausgebildet ist, zugeleitet und durchläuft das System von unten nach oben.

Der Mischpumpe der unteren Stufe führt man neben der benzolischen Lösung der Perpropionsäure pro Stunde 67 ml einer wässrigen Lösung zu, die man erhält, wenn man die Wasserphase des Kopfproduktes der nachfolgenden Azeotropdestillation (Destillationseinheit (16)), die in einer Menge von 60 ml pro Stunde anfällt und 1,48 Gew.-% $H_2O_2$, 2,43 Gew.-% Perpropionsäure und 0,27% Propionsäure enthält, mit 7 ml entionisiertem Wasser mischt. Die benzolische Lösung, die dem unteren Scheidegefäss als leichte Phase entnommen wird, wird nach dem Durchlaufen der mittleren Mischer-Scheider-Anordnung, zusammen mit 17 ml/h Frischwasser der zur oberen Mischer-Scheider-Einheit gehörenden Mischpumpe zugeführt. Die nach erfolgter Phasentrennung hier anfallende wässrige Phase wird in die mittlere Extraktionsstufe geleitet. Die in dem mittleren und unteren Scheidegefäss sich als schwere Phase sammelnden wässrigen Lösungen werden vereinigt und über (14) wieder in die Extraktionseinheit (5) eingegeben, derart, dass man diesen Strom, bestehend aus einer wässrigen Lösung, die 25,23 Gew.-% Perpropionsäure, 6,8 Gew.-% Wasserstoffperoxid und 22,35 Gew.-% Propionsäure enthält, mit einer Menge von 81 ml/h unmittelbar vor Eintritt in die pulsierte Siebbodenkolonne (Extraktionssystem (5)), mit dem aus dem Reaktionssystem (1) kommenden Produktstrom (4) mischt.

Aus dem zur oberen Mischer-Scheider-Einheit gehörenden Scheidegefäss des Extraktionssystems (12) werden über Leitung (15) als leichte Phase in der Stunde 1493 g (= 1570 ml) einer benzolischen Lösung von Perpropionsäure mit der Zusammensetzung 20,04 Gew.-% Perpropionsäure, 11,41 Gew.-% Propionsäure, 3,95 Gew.-% Wasser und 0,2 Gew.-% Wasserstoffperoxid abgezogen und in die Destillationseinheit (16) eingegeben, wo die Lösung azeotrop getrocknet wird. Vor Eingabe in die Destillationseinheit (16) wird die benzolische Lösung der Perpropionsäure pro Stunde mit 5 ml einer etwa 3 gew.-%igen Lösung eines Stabilisators vom Typ der im Handel erhältlichen Na-Salze von partiell veresterten Polyphosphorsäuren in Propionsäure versetzt.

Die Destillationseinheit (16) wird bei 210 Torr betrieben und besteht aus einem Dünnschichtverdampfer, einer 50 cm langen, mit 5 Glockenböden versehenen Kolonne von 50 mm Durchmesser, einem Kondensator, sowie einem Abscheider zur Phasentrennung des Destillats am Kopf der Kolonne. Die Temperatur im Sumpf der Kolonne beträgt 65°C. Als Destillat werden pro Stunde 60 ml Wasser und ca. 915 ml Benzol erhalten. Das Benzol wird als Rücklauf auf die Kolonne gegeben, während man das im Abscheider anfallende Wasser, wie bereits beschrieben, als Waschwasser über (35) in die untere Stufe der Extraktionseinheit (12) eingibt. Als Sumpfprodukt dieser Azeotropdestillation erhält man mit einer Menge von 1438 g pro Stunde eine 20,71 gew.-%ige benzolische Lösung von Perpropionsäure, die ausserdem 12,18 Gew.-% Propionsäure, sowie 0,1 Gew.-% Wasser und 0,15 Gew.-% Wasserstoffperoxid enthält.

Die Ausbeute an Perpropionsäure in dem so getrockneten benzolischen Extrakt beträgt 96,15% bezogen auf das in das Verfahren eingesetzte Wasserstoffperoxid.

Die so erhaltene, getrocknete benzolische Lösung von Perpropionsäure wird mit überschüssigem Butadien in einer vierstufigen Kesselkaskade (Reaktionssystem (18)) umgesetzt. Die Reaktion wird bei einem Druck von 2 bar durchgeführt. Das Butadien wird gasförmig über die Leitungen (22), (20) und (19) in den ersten Reaktor eingegeben. Der Überschuss an Butadien, bezogen auf in die Reaktion eingesetzte Perpropionsäure, beträgt insgesamt 170 Mol.% (= 482 g Butadien). Der erste Reaktor dieser vierstufigen Kaskade, der ebenso wie die drei nachfolgenden Reaktionsgefässe mit einer Rührvorrichtung versehen ist und einen Inhalt von 1600 ml besitzt, wird bei einer Temperatur von 35°C, der zweite, dritte und vierte Reaktor bei einer Temperatur von jeweils 40°C betrieben.

Die eingesetzte Perpropionsäure wird unter diesen Reaktionsbedingungen zu 99,8% umgesetzt. Hinter dem vierten Reaktor wird das Reaktionsgemisch, das mit einer Menge 1920 g pro Stunde anfällt und im Mittel die Zusammensetzung von 15,62 Gew.-% Butadien, 11,67 Gew.-% Vinyloxiran, 21,46 Gew.-% Propionsäure und 50,08 Gew.-% Benzol sowie 0,13 Gew.-% Wasser besitzt, nach Abkühlen auf 30°C im Abscheider (21) auf Normaldruck entspannt, wobei ein geringer Teil des Butadiens (37 g/h) ausgast, das über (22) dem Reaktionssystem (18) wiederzugeführt wird. Das nach dem Entspannen erhaltene Gemisch wird nunmehr, nachdem es mit 2,0 g 4-tert.-Butyl-benzkatechin versetzt worden ist, einer ersten Destillationskolonne (25) zugeführt, wo man das gesamte Vinyloxiran zusammen mit dem Butadien und einem Teil des Benzols als Destillat abnimmt. Dieses Destillat, das im Mittel 32,6 Gew.-% Butadien, 27,25 Gew.-% Vinyloxiran, 39,9 Gew.-% Benzol und geringe Mengen Wasser enthält und mit einer Menge von 822 g pro Stunde anfällt, führt man der Destillationskolonne (27) über (26) zu, wo in der Stunde 224 g eines 99,9%igen Vinyloxirans (Produktstrom 28) sowie 268 g Butadien erhalten werden, das über Leitung (20) in das Reaktionssystem (18) geführt wird. Die Sumpfprodukte der Kolonnen (25) und (27) werden über Leitung (29) bzw. (30) der Kolonne (31) zugeführt, wo das Benzol mit einer Menge von 961 g/h als Kopfprodukt wiedergewonnen und danach über Leitung (6) in das Extraktionssystem (5) zurückgefördert wird. Der Sumpf der Kolonne (31) gelangt über Leitung (32) in die Destillationskolonne (33). Als Kopfprodukt erhält man hier in der Stunde 415 g/h an Propionsäure, die über Leitung (3) in das Reaktionssystem (1) zurückgeführt werden. Aus dem Sumpf der Kolonne (33) trägt man über (34) in der Stunde 15 g eines Gemisches von höhersiedenden Verbindungen aus, in dem im wesentlichen Butan-(1)-diol-(3,4)-mono- und dipropionat enthalten sind.

Die Ausbeute von Vinyloxiran beträgt somit, bezogen auf die in das Reaktionssystem (18) eingesetzte Perpropionsäure, 96,7% oder, bezogen auf in das Verfahren bei (8) über (9) eingesetztes Wasserstoffperoxid, 93%.

**Patentansprüche**

1. Verfahren zur Herstellung von Vinyloxiran aus Wasserstoffperoxid und Butadien, dadurch gekennzeichnet, dass man

a) eine 10 bis 45 Gew.-% eines wasserlöslichen, sauren Katalysator und 20 bis 50 Gew.-% Wasserstoffperoxid enthaltende wässrige Lösung mit einer 2 bis 5 Kohlenstoffatomen enthaltenden Carbonsäure im Molverhältnis Wasserstoffperoxid zu Carbonsäure wie 0,5–30:1 bei Temperaturen von 10 bis 70°C umsetzt,

b) das erhaltene Reaktionsgemisch mit einem inerten, organischen Lösungsmittel im Gegenstrom extrahiert,

c) das im wesentlichen Wasserstoffperoxid und sauren Katalysator enthaltende wässrige Raffinat der Extraktion ganz oder teilweise durch destillative Entfernung von Wasser aufkonzentriert,

d) das aufkonzentrierte Raffinat sowie den gegebenenfalls nicht aufkonzentrierten Anteil des Raffinats unter Wiederherstellung der für die Umsetzung mit der Carbonsäure erforderlichen Konzentrationen an Wasserstoffperoxid und wasserlöslichem sauren Katalysator in die Umsetzungsstufe (a) zurückführt, wobei das zur Wiederherstellung der für die Umsetzung mit der Carbonsäure erforderlichen Wasserstoffperoxidkonzentration benötigte Wasserstoffperoxid dem aufzukonzentrierenden Anteil des Raffinats vor oder nach der destillativen Entfernung von Wasser gemäss (c) zugesetzt wird oder zu dem gegebenenfalls nicht aufkonzentrierten Teil des Raffinats zugegeben wird,

e) den im wesentlichen Percarbonsäure und Carbonsäure enthaltenden organischen Extrakt mit Wasser oder einer wässrigen Lösung behandelt und

f) den nunmehr praktisch wasserstoffperoxidfreien, wasserhaltigen, organischen Extrakt einer Azeotropdestillation derart unterwirft, dass der Restgehalt an Wasser im Sumpfprodukt der Azeotropkolonne weniger als 0,5 Gew.-% beträgt,

g) die nunmehr gewonnene, Percarbonsäure und Carbonsäure enthaltende, organische Lösung mit Butadien im Überschuss bei einem Molverhältnis von Butadien zu Percarbonsäure von 1,5 bis 6:1 bei Temperaturen von 0°C bis 80°C und bei einem Druck von 0,8 bar bis 20 bar umsetzt, und

h) das vinyloxiranhaltige Reaktionsgemisch auf destillativem Wege aufarbeitet, wobei reines Vinyloxiran isoliert wird und das überschüssige Butadien, die Carbonsäure und das inerte, organische Lösungsmittel wiedergewonnen und ganz oder teilweise in das Verfahren zurückgeführt werden.

2. Verfahren nach Anspruch 1, dadurch gekennzeichnet, dass man in Stufe (a) eine 20 bis 43 Gew.-% Schwefelsäure und 22 bis 35 Gew.-% Wasserstoffperoxid enthaltende wässrige Lösung einsetzt.

3. Verfahren nach Anspruch 1 und 2, dadurch gekennzeichnet, dass man in Stufe (a) Wasserstoffperoxid und Carbonsäure in einem Molverhältnis von 0,5 bis 1,3 oder 3,5 bis 25:1 einsetzt.

4. Verfahren nach Anspruch 1 bis 3, dadurch gekennzeichnet, dass in Stufe (a) als Carbonsäure Essigsäure, Propionsäure, n-Buttersäure oder Isobuttersäure zur Umsetzung gebracht wird.

5. Verfahren nach Anspruch 1 bis 4, dadurch gekennzeichnet, dass in Stufe (b) als inertes, organisches Lösungsmittel 1,2-Dichlorpropan, Chlorbenzol, Toluol, Benzol, n-Butylacetat oder Diisopropyläther eingesetzt wird.

6. Verfahren nach Anspruch 1 bis 5, dadurch gekennzeichnet, dass man in Stufe (b) das Mengenverhältnis von inertem, organischen Lösungsmittel zu dem gemäss Stufe (a) gewonnenen, die Percarbonsäure enthaltenden Reaktionsgemisch wie 4 bis 0,3:1 wählt.

7. Verfahren nach Anspruch 1 bis 6, dadurch gekennzeichnet, dass man in Stufe (c) die destillative Entfernung von Wasser bei Drücken von 40 bis 150 Torr und bei Temperaturen von 60 bis 85°C vornimmt.

8. Verfahren nach Anspruch 1 bis 7, dadurch gekennzeichnet, dass man den wasserstoffperoxid- und schwefelsäurehaltigen Seitenstrom einer Regenerierungsstufe zuführt und gegebenenfalls die wiedergewonnenen Anteile an Wasserstoffperoxid und Schwefelsäure wieder in das Verfahren eingibt.

9. Verfahren nach Anspruch 1 bis 8, dadurch gekennzeichnet, dass in Stufe (g) die Umsetzung bei einem Molverhältnis von Butadien zu Percarbonsäure von 1,5 bis 4:1 durchgeführt wird.

10. Verfahren nach Anspruch 1 bis 9, dadurch gekennzeichnet, dass das in Stufe (h) gewonnene organische Lösungsmittel in Stufe (b), die in Stufe (h) gewonnene Carbonsäure in Stufe (a) und das in Stufe (h) gewonnene Butadien in Stufe (g) wieder zurückgeführt werden.

**Claims**

1. Process for the preparation of vinyloxirane from hydrogen peroxide and butadiene, characterised in that

a) an aqueous solution containing 10 to 45% by weight of a watersoluble, acid catalyst and 20 to 50% by weight of hydrogen peroxide is reacted with a carboxylic acid containing 2 to 5 carbon atoms in a molar ratio of hydrogen peroxide to carboxylic acid of 0.5–30:1 at temperatures of 10 to 70°C,

b) the resulting reaction mixture is extracted with an inert, organic solvent, in counter-current,

c) all or some of the aqueous raffinate from the extraction, which essentially contains hydrogen peroxide and acid catalyst, is concentrated by removing the water by distillation,

d) the concentrated raffinate, and the portion of the raffinate which is optionally not concentrated, are recycled into the reaction stage (a), the concentrations of hydrogen peroxide and water - soluble acid catalyst required for the reaction with the carboxylic acid being re-established, and the hydrogen peroxide necessary for re-establishing the hydrogen peroxide concentration required for the reaction with the carboxylic acid being added, before or after the removal of water by distillation according to (c), to the portion of the raffinate to be concentrated or to the portion of the raffinate which is optionally not concentrated,

e) the organic extract, which essentially contains percarboxylic acid and carboxylic acid, is treated with water or an aqueous solution,

f) the water-containing organic extract, which is now virtually free from hydrogen peroxide, is subjected to azeotropic distillation in a manner such that the residual content of water in the bottom product of the azeotropic column is less than 0.5% by weight,

g) the organic solution now obtained, which contains percarboxylic acid and carboxylic acid, is reacted with butadiene in excess at a molar ratio of butadiene to percarboxylic acid of 1.5 to 6:1 at temperatures of 0°C to 80°C and under a pressure of 0.8 to 20 bars, and

h) the vinyloxirane-containing reaction mixture is worked up by a distillative route, pure vinyloxirane being isolated and the excess butadiene, the carboxylic acid and the inert organic solvent being recovered and wholly or partly recycled into the process.

2. Process according to Claim 1, characterised in that an aqueous solution containing 20 to 43% by weight of sulphuric acid and 22 to 35% by weight of hydrogen peroxide is employed in stage (a).

3. Process according to Claim 1 and 2, characterised in that hydrogen peroxide and carboxylic

acid are employed in a molar ratio of 0.5 to 1.3 or 3.5 to 25:1 in stage (a).

4. Process according to Claim 1 to 3, characterised in that acetic acid, propionic acid, n-butyric acid or isobutyric acid is reacted as the carboxylic acid in stage (a).

5. Process according to Claim 1 to 4, characterised in that 1,2-dichloropropane, chlorobenzene, toluene, benzene, n-butyl acetate or diisopropyl ether is employed as the inert, organic solvent in stage (b).

6. Process according to Claim 1 to 5, characterised in that the ratio of inert, organic solvent to the reaction mixture, which is obtained according to stage (a) and contains percarboxylic acid of 4 to 0.3:1 is chosen in stage (b).

7. Process according to Claim 1 to 6, characterised in that the removal of water by distillation in stage (c) is carried out under pressures of 40 to 150 mm Hg and at temperatures of 60 to 85°C.

8. Process according to Claim 1 to 7, characterised in that the side stream containing hydrogen peroxide and sulphuric acid is fed to a regenerating stage and the portions of hydrogen peroxide and sulphuric acid recovered are optionally reintroduced into the process.

9. Process according to Claim 1 to 8, characterised in that the reaction in stage (g) is carried out at a molar ratio of butadiene to percarboxylic acid of 1.5 to 4:1.

10. Process according to Claim 1 to 9, characterised in that the organic solvent obtained in stage (h) is recycled into stage (b), the carboxylic acid obtained in stage (h) is recycled into stage (a) and the butadiene obtained in stage (h) is recycled into stage (g).

**Revendications**

1. Procédé de préparation du vinyloxirane à partir du peroxyde d'hydrogène et du butadiène, caractérisé en ce que

a) on fait réagir une solution aqueuse contenant de 10 à 45% en poids d'un catalyseur acide hydrosoluble et de 20 à 50% en poids de peroxyde d'hydrogène avec un acide carboxylique contenant de 2 à 5 atomes de carbone dans un rapport molaire peroxyde d'hydrogène/acide carboxylique de 0,5 à 30:1, à des températures de 10 à 70°C,

b) on extrait le mélange de réaction obtenu à contre-courant par un solvant organique inerte,

c) on concentre en totalité ou en partie, par élimination de l'eau par distillation, le raffinat aqueux de l'extraction contenant essentiellement du peroxyde d'hydrogène et le catalyseur acide,

d) on recycle le raffinat concentré et la partie éventuellement non concentrée du raffinat en rétablissant les concentrations en peroxyde d'hydrogène et catalyseur acide hydrosoluble nécessaires pour la réaction avec l'acide carboxylique au stade de réaction (a), le peroxyde d'hydrogène nécessaire pour le rétablissement de la concentration en peroxyde d'hydrogène exigée pour la réaction avec l'acide carboxylique étant ajouté à la partie à concentrer du raffinat avant ou après l'élimination de l'eau par distillation selon (c) ou à la partie du raffinat éventuellement non concentrée,

e) on traite l'extrait organique contenant essentiellement l'acide percarboxylique et l'acide carboxylique par l'eau ou une solution aqueuse et,

f) on soumet l'extrait organique aqueux maintenant pratiquement exempt de peroxyde d'hydrogène à une distillation azéotropique dans des conditions telles que la teneur résiduelle en humidité dans le produit de pied de la colonne azéotropique soit inférieure à 0,5% en poids,

g) on fait réagir la solution organique alors obtenue, contenant de l'acide percarboxylique et l'acide carboxylique avec du butadiène en excès, à un rapport molaire butadiène/acide percarboxylique de 1,5 à 6:1, à des températures de 0 à 80°C et sous une pression de 0,8 bar à 20 bars et,

h) on traite le mélange de réaction contenant le vinyloxirane par distillation en isolant le vinyloxirane pur, en récupérant l'excès de butadiène, l'acide carboxylique et le solvant organique inerte et en les recyclant en totalité ou en partie dans l'opération.

2. Procédé selon la revendication 1, caractérisé en ce que, au stade (a), on utilise une solution aqueuse contenant de 20 à 43% en poids d'acide sulfurique et de 22 à 35% en poids de peroxyde hydrogène.

3. Procédé selon la revendication 1 et 2, caractérisé en ce que l'on utilise au stade (a) le peroxyde d'hydrogène et l'acide carboxylique dans un rapport molaire de 0,5 à 1,3 ou 3,5 à 25:1.

4. Procédé selon les revendications 1 à 3, caractérisé en ce que, au stade (a), l'acide carboxylique utilisé pour la réaction est l'acide acétique, l'acide propionique, l'acide n-butyrique ou l'acide iso-butyrique.

5. Procédé selon les revendications 1 à 4, caractérisé en ce que, au stade (b), on utilise en tant que solvant organique inerte le 1,2-dichloropropane, le chlorobenzène, le toluène, le benzène, l'acétate de n-butyle ou l'éther diisopropylique.

6. Procédé selon les revendications 1 à 5, caractérisé en ce que, au stade (b), on opère avec des proportions relatives de 4 à 0,3:1 entre le solvant organique inerte et le mélange de réaction contenant l'acide percarboxylique obtenu au stade (a).

7. Procédé selon les revendications 1 à 6, caractérisé en ce que, au stade (c), l'élimination de l'eau par distillation est effectuée sous des pressions de 40 à 150 mmHg et à des températures de 60 à 85°C.

8. Procédé selon les revendications 1 à 7, caractérisé en ce que l'on envoie le courant dévié contenant du peroxyde d'hydrogène et de l'acide sulfurique à un stade de régénération et le cas échéant on retourne dans le procédé les fractions récupérées de peroxyde d'hydrogène et d'acide sulfurique.

9. Procédé selon les revendications 1 à 8, caractérisé en ce que, au stade (g), on effectue la réaction à un rapport molaire butadiène/acide

percarboxylique de 1,5 à 4:1.

10. Procédé selon les revendications 1 à 9, caractérisé en ce que l'on recycle au stade (b) le solvant organique obtenu au stade (h), au stade (a) l'acide carboxylique obtenu au stade (h) et au stade (g) le butadiène obtenu au stade (h).

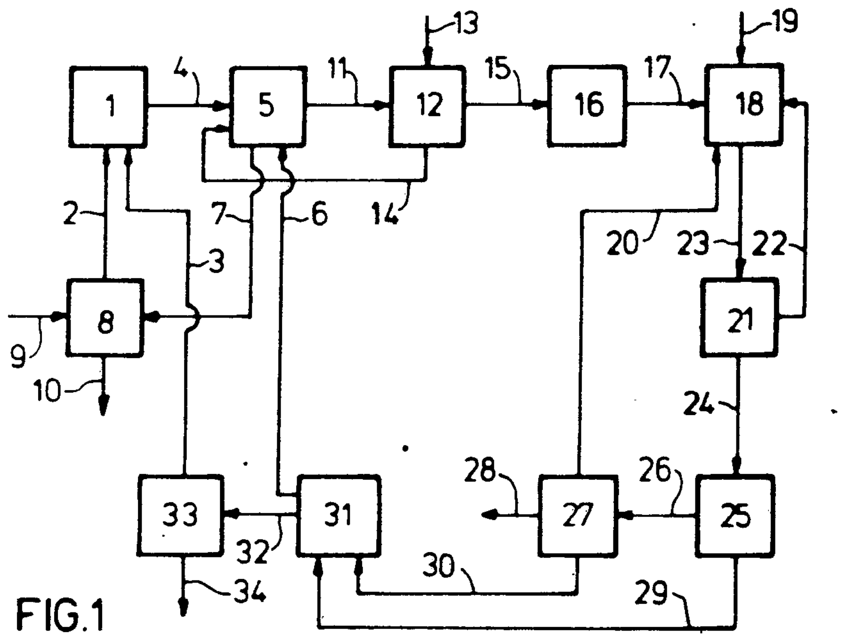
1
5
12
16
18
8
21
33
31
27
25
2
3
4
6
7
9
10
11
13
14
15
17
19
20
22
23
24
26
28
29
30
32
34

FIG.1

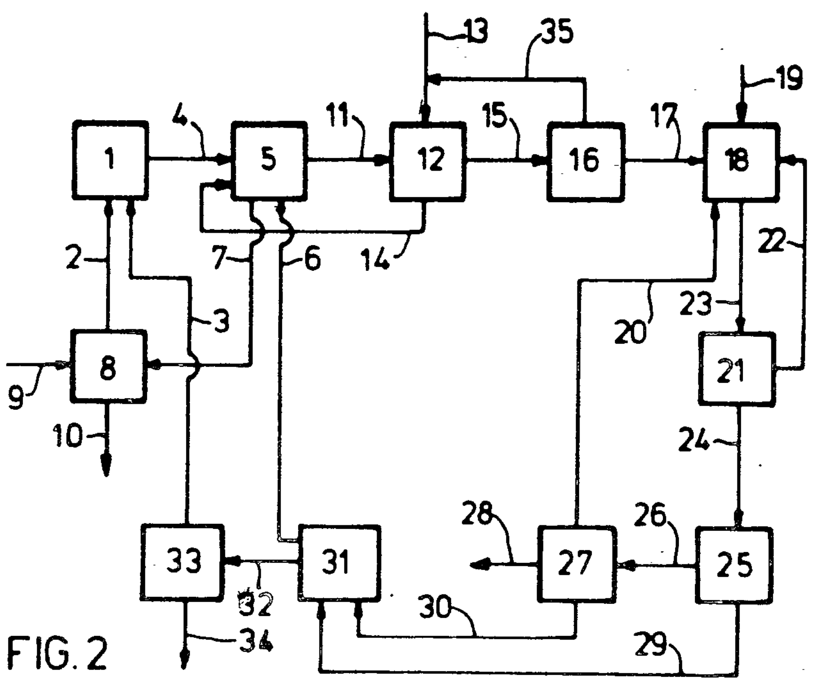
1
5
12
16
18
8
21
33
31
27
25
2
3
4
6
7
9
10
11
13
14
15
17
19
20
22
23
24
26
28
29
30
32
34
35

FIG. 2